# FASCICULE DE BREVET EUROPEEN

(11) **EP 1 153 033 B1**
(45) Date de publication et mention de la délivrance du brevet: **02.04.2003**
(21) Numéro de dépôt: 00902710.3
(22) Date de dépôt: 07.02.2000
(51) Int. Cl.: C07H 17/065, A61K 7/00, A61K 35/78, A23L 1/00

(54) **PROCEDE DE PURIFICATION D'UN EXTRAIT DE FRUIT ROUGE CONTENANT DES ANTHOCYANOSIDES**
VERFAHREN ZUR HERSTELLUNG VON ANTHOCYANOSID-ENTHALTENDEN EXTRAKTEN VON ROTEN FRÜCHTEN
METHOD FOR PURIFYING A RED FRUIT EXTRACT CONTAINING ANTHOCYANOSIDES

(30) Priorité: 15.02.1999 FR 9901959
(43) Date de publication de la demande: 14.11.2001
(73) Titulaire: Ferlux (Société Anonyme), 63800 Cournon d'Auvergne (FR)
(72) Inventeur: SOULIER, Chrystèle, F-63960 Veyre Monton (FR); DUFOUR, Dominique, F-63100 Beaumont (FR)
(74) Mandataire: Thivillier, Patrick
(86) Numéro de dépôt international: FR0000275
(87) Numéro de publication internationale: WO00047596

(56) Documents cités:
- EP-A- 0 412 300
- FR-A- 2 299 385
- FR-A- 2 456 747
- FR-A- 2 641 283
- GB-A- 1 235 379

## Description

L'invention se rapporte à un procédé de purification d'un extrait de fruit rouge contenant des anthocyanosides. Elle concerne également l'extrait obtenu par ledit procédé de même que son utilisation.

Dans la suite de la description et dans les revendications, on désigne par « fruit rouge », notamment les baies de sureau (*Sambucus nigra*), les baies de cassis (*Ribes nigrum*), les groseilles (*Ribes rubrum*), les mûres (*Morus nigra*), le raisin (*Vitis vinifera*), et plus particulièrement les myrtilles (*Vaccinium myrtillus* et autres espèces du genre *Vaccinium),* et ce, sans que ce soit limitatif.

De façon générale, on désigne par "anthocyanosides", un hétéroside résultant de la combinaison d'une génine anthocyanique avec un ou plusieurs groupements osidiques. Ces anthocyanosides existent sous forme de monomères, mais aussi de dimères, d'oligomères et de polymères suivant un degré de polymérisation croissant. La liaison anthocyanoside- anthocyanoside s'effectue entre le C4 du noyau C et le C8 du noyau A.

Dans le cas présent, les anthocyanosides désignent les structures sous forme de monomères, dont le poids moléculaire varie de 400 à 600 Daltons. Certaines formes de dimères peuvent également être présentes, dont le poids moléculaire peut atteindre environ 1 000 Daltons.

Les fruits rouges sont riches de pigments appelés anthocyanosides, dont on a prouvé qu'ils avaient un effet sur la microcirculation sanguine, en agissant notamment en tant que facteur vitaminique P, en tant qu'agent antioxydant, antiagrégant plaquettaire et antiradicalaire. Ces propriétés ont été reconnues pour le traitement des troubles impliquant la circulation rétinienne, dans le traitement des troubles fonctionnels de la fragilité capillaire et des troubles visuels d'origine vasculaire. Ces propriétés ont également été mises en oeuvre pour le traitement de l'hespéranopie et de la myopie, dans les spécialités pharmaceutiques commercialisées sous les marques DIFRAREL® 100 et DIFRAREL® E par les laboratoires LEURQUIN MEDIOLANUM.

Plusieurs procédés d'extraction d'anthocyanosides à partir de plantes ou parties de plantes ont été proposés.

Le document FR-A-2 299 385 décrit ainsi un procédé d'extraction d'anthocyanes à partir de marc de raisin comprenant une étape d'extraction proprement dite, puis une étape de concentration de l'extrait obtenu.

Selon ce procédé, l'étape d'extraction consiste à traiter les marcs par une solution d'extraction aqueuse acide (pH = 2) additionnée de SO₂ à chaud (entre 40 et 55° C). La solution limpide obtenue, contenant les anthocyanosides, mais également les acides, les sels, les polyphénols et les protéines, est ensuite concentrée. Pour ce faire, elle est chargée sur une résine. La résine est ensuite éluée avec une solution d'elution contenant au choix une cétone, une amide ou une solution aqueuse d'un hydroxyde alcalin ou alcalino-terreux. Les anthocyanes sont enfin séparés de l'éluat obtenu.

Il ressort des exemples décrits, notamment de l'exemple 1, qu'à partir d'un extrait contenant 120 mg d'anthocyanes par litre, on obtient en sortie de résine un extrait comprenant 800 mg par litre d'anthocyanes, c'est-à-dire un extrait 6,6 fois plus concentré.

Toutefois, même si le procédé mis en oeuvre permet d'augmenter notablement la concentration d'anthocyanosides dans l'extrait, cette concentration ne présage en rien d'une purification des anthocyanosides.

De plus et surtout, l'extraction initiale des anthocyanosides par un solvant additionné de SO₂ conduit à la fixation des anthocyanosides sur la résine sous forme modifiée, susceptible donc de perturber les caractéristiques physico-chimiques de l'anthocyanoside et partant, son activité.

Plus récemment, on a proposé dans le document EP-A-412 300, un procédé d'extraction puis de purification d'anthocyanosides à partir de myrtilles.

L'étape d'extraction proprement dite consiste à mettre des fruits congelés au contact d'une solution aqueuse de méthanol, chaque extraction s'étendant sur une période de quatre heures.

L'extrait obtenu est ensuite purifié. Pour ce faire, il est tout d'abord concentré sous vide, le concentrât résultant étant ensuite additionné de bisulfite de sodium. Il se forme alors une liaison entre les anthocyanosides et les ions bisulfite. Après trois heures d'agitation et neutralisation par addition d'une solution d'hydroxyde de sodium, on charge l'extrait obtenu sur une colonne d'une résine polymère non ionogène puis on élue la colonne à l'aide d'eau purifiée. L'éluat est alors acidifié à pH = 1 par de l'acide chlorhydrique (HCl) concentré. Pour éliminer le SO₂, on fait ensuite barboter dans la solution obtenue de l'azote, de sorte à dissocier le complexe anthocyanosides-bisulfite. Cette dissociation conduit à la libération de dioxyde de soufre. La solution aqueuse est ensuite extraite à l'aide de butanol. La solution butanolique est additionnée de 14 volumes d'acétate d'éthyl. Après une nuit de repos, le précipité est séché à 40° C.

De même que précédemment, le procédé mis en oeuvre nécessite toujours l'utilisation d'ions bisulfite conduisant à la formation d'un complexe anthocyanoside bisulfite, susceptible d'altérer les propriétés physico-chimiques des anthocyanosides recherchés.

De plus, le recours à l'azote pour régénérer les anthocyanosides entraîne des dégagements de dioxyde de soufre, susceptibles de créer des problèmes environnementaux et de lourds traitements des rejets.

Il est enfin également important de souligner la multitude de solvants utilisés (méthanol, butanol, acétate d'éthyl) et les traitements chimiques lourds que ce procédé d'extraction implique : utilisation de SO₂, NaOH, acidification à pH = 1 par HCI concentré et utilisation d'azote.

Par le brevet FR 2641983, on connaît un procédé d'extraction de substances colorantes et plus particulièrement un procédé d'extraction des anthocyanosides des baies de fruits, de myrtilles, de cassis ou d'airelles nécessitant notamment l'utilisation d'un solvant super critique faiblement polaire que l'on distille ultérieurement. Le résidu d'extraction est purifié par les moyens physiques usuels notamment par chromatographie sur une colonne de polyamide puis élution au méthanol chlorydrique.
Les anthocyanosides ainsi obtenus et notamment ceux de la myrtille sont des principes actifs de médicaments.

A partir de cet état de la technique , l'invention concerne un procédé de purification d'un extrait de fruits rouges contenant des anthocyanosides susceptibles de résoudre les problèmes suivants :
- s'affranchir de l'utilisation de tout additif du type SO₂ susceptible de modifier la structure et les caractéristiques physico-chimiques des anthocyanosides extraits ;
- éviter tout dégagement de substances nocives et notamment de dioxyde de soufre ;
- augmenter la concentration d'anthocyanosides de l'extrait purifié obtenu ;
- réaliser une purification sans modification structurelle des anthocyanosides ;
- réduire au maximum la quantité de solvants résiduels contenus dans l'extrait purifié ;
- obtenir un extrait dans lequel les solvants résiduels sont clairement identifiés, et dont la faible quantité répond aux indications ICH (International Conference on Harmonisation of Technical Requirements for Registration of Pharmaceuticals for Human Use).

Pour ce faire, l'invention a pour objet un procédé de purification d'un extrait de fruit rouge contenant des anthocyanosides selon lequel :
- on reprend ledit extrait avec une solution aqueuse ;
- on refroidit ensuite l'extrait aqueux jusqu'à ce qu'il atteigne une température homogène inférieure à 15° C ;
- après filtration de l'extrait aqueux, on récupère le perméat obtenu que l'on charge sur une résine polymérique macroréticulée ;
- on rince ensuite la résine par de l'eau déminéralisée ;
- puis, on élue la résine obtenue avec une solution d'élution alcoolique ;
- enfin, on concentre puis on sèche l'éluat obtenu.

De la sorte, en s'affranchissant de tout solvant additionné de SO₂, le procédé de purification de l'invention conduit dans des conditions douces, n'entraîne aucune modification des caractéristiques physico-chimiques natives, donc aucune dénaturation des anthocyanosides extraits. Cette caractéristique peut être confirmée par la superposition des profils chromatographiques HPLC en fonction du temps de rétention de l'extrait d'une part et du fruit rouge de départ d'autre part.

De plus, on observe que la combinaison des différentes étapes du procédé, et notamment l'étape de refroidissement suivie de la filtration et du passage sur résine, conduit, de façon tout à fait surprenante, à augmenter considérablement le titre d'anthocyanosides extrait dans l'extrait sec final. Pour une température de l'extrait aqueux supérieure à 15° C, on n'obtient pas une purification suffisante des anthocyanosides.

Dans une première forme de réalisation de l'invention, le procédé de purification est effectué sur un extrait alcoolique de fruit rouge obtenu selon le procédé suivant :
- on sépare tout d'abord la pulpe des fruits rouges entiers ;
- on met ensuite ladite pulpe au contact d'une solution d'extraction alcoolique ;
- puis, on sépare la phase solide de la phase liquide ;
- enfin, on évapore sous vide la majeure partie de l'alcool résiduel contenu dans la phase liquide, afin d'obtenir un concentrât alcoolique.

Avantageusement, le solvant utilisé pour l'extraction alcoolique est choisi dans le groupe comprenant le méthanol, l'éthanol, le butanol et l'acétone.

En pratique, l'extraction alcoolique est effectuée à température ambiante en au moins deux étapes successives, chacune étant d'une durée de 20 minutes. Le solvant est ensuite évaporé. En outre, on peut prévoir également d'effectuer l'extraction des anthocyanosides non pas à partir de la seule pulpe, mais à partir des fruits entiers.

De plus, la séparation phase solide/phase liquide peut être effectuée par tout moyen connu, notamment centrifugation.

Selon une seconde forme de réalisation de l'invention, le procédé de purification est effectué à partir d'extraits de fruits rouges disponibles dans le commerce ou d'extrait d'anthocyanosides prépurifiés, présentés chacun sous forme liquide ou de poudre. Dans ce cas, l'extrait de fruit ou l'extrait prépurifié peut alors être repris avant l'étape de purification, au choix par de l'alcool, notamment du méthanol, ou de l'eau.

Dans le procédé de purification de l'invention, le refroidissement de l'extrait de fruit rouge est effectué avantageusement jusqu'à ce que la température dudit extrait soit homogène et inférieure à 10° C, de préférence inférieure à 5° C, cette température étant maintenue pendant au moins douze heures.

Concernant l'étape de filtration de l'extrait aqueux, celle-ci peut être effectuée sur filtre cellulose, toile inox avec un seuil de coupure compris entre 0 et 100 micromètres ou équivalent.

Comme déjà dit, le perméat issu de l'étape de filtration est ensuite chargé sur une résine macroréticulée adsorbante.

Par ailleurs, afin d'augmenter d'avantage le titre et la concentration d'anthocyanosides dans l'extrait final, la solution alcoolique avec laquelle on élue les anthocyanosides de la résine est préférentiellement une solution aqueuse d'éthanol dont la concentration en éthanol est comprise entre 10 et 90 %, avantageusement voisine de 40 %.

L'éluat obtenu est concentré sous température contrôlée aux environs de 30° C, puis séché par lyophilisation ou encore atomisation de sorte à obtenir une poudre.

On observe par ailleurs que la poudre obtenue contient une quantité résiduelle de solvant alcoolique très faible, inférieure à 3 000 ppm pour le méthanol, (si la purification est effectuée à partir d'un extrait méthanolique) et inférieur à 2000 ppm pour l'éthanol. Ces solvants résiduels sont identifiés par CPG et sont les seuls contenus dans la poudre finale.

L'invention se rapporte également à l'extrait susceptible d'être obtenu par le procédé de purification ci-avant décrit.

En outre, par son titre élevé en anthocyanosides originels et sa faible teneur en solvants résiduels, l'extrait pourra être utilisé dans toute composition cosmétique, pharmaceutique et diététique. Il pourra également être utilisé dans les compositions nutraceutiques, les alicaments, les aliments enrichis et les compléments alimentaires.

L'invention et les avantages qui en découlent ressortiront mieux des exemples de réalisation suivants, à l'appui des figures annexées dans lesquelles :
- la figure 1 est une comparaison d'un chromatogramme HPLC de l'extrait final obtenu selon l'exemple 1 par rapport à celui du fruit et celui de la pulpe d'origine ;
- la figure 2 est une comparaison identique effectuée à partir de l'extrait obtenu selon l'exemple 2.

Les deux exemples qui suivent se rapportent à l'extraction d'anthocyanosides à partir de pulpe de myrtille.

### Exemple 1

### 1/ Séparation de la pulpe

On presse 125 kilos de myrtilles originaire de Lithuanie par tout moyen connu. A l'issue du pressage, on sépare la pulpe représentant 52,24 kilos et le jus représentant 71,48 kilos.

### 2/ Extraction

On procède ensuite à deux extractions successives à partir de 35 kilos de pulpe de myrtilles avec chaque fois 210 litres de méthanol (soit 1 volume de pulpe pour 6 volumes de solution d'extraction) pendant vingt minutes à température ambiante. En fin d'extraction, on presse la pulpe de sorte à récupérer la fraction méthanolique. On évapore ensuite le méthanol sous vide, la température de l'extrait étant égale alors à 22° C.

### 3/Purification

On reprend ensuite le concentrât avec 70 litres d'eau déminéralisée (soit un volume de pulpe pour 2 volumes d'eau).

L'extrait aqueux ainsi obtenu est ensuite stocké à une température inférieure à 15° C pendant une nuit.

On procède ensuite à la filtration de la solution sur une toile inox 50 micromètres.

Puis on charge une résine macroréticulée, par exemple la résine XAD761 commercialisée par ROHM & HAAS avec l'extrait aqueux. On utilise en pratique 25 litres de résine.

Une fois la résine chargée, on la rince avec 105 litres d'eau déminéralisée (soit un volume de pulpe pour 3 volumes d'eau déminéralisée).

On procède ensuite à l'élution de la résine par 200 litres d'éthanol à 40 %.

On concentre ensuite l'éluat sous vide, la température de l'extrait étant alors comprise entre 26 et 31° C.

Pour obtenir une poudre, on atomise ensuite l'éluat.

La teneur en anthocyanosides de l'extrait final est égale à 50.46 % du poids final soit 53.50 % en poids de matière sèche.

Ces résultats sont exprimés par rapport au chlorure de cyanidine, étalon considéré pour l'analyse HPLC, référence 0909S, Fournisseur : Extrasynthèse.

On note que la quantité résiduelle de solvant dans l'extrait final est :
- 0 ppm pour le butanol,
- inférieure à 3000 ppm pour le méthanol,
- inférieure à 2000 ppm pour l'éthanol.

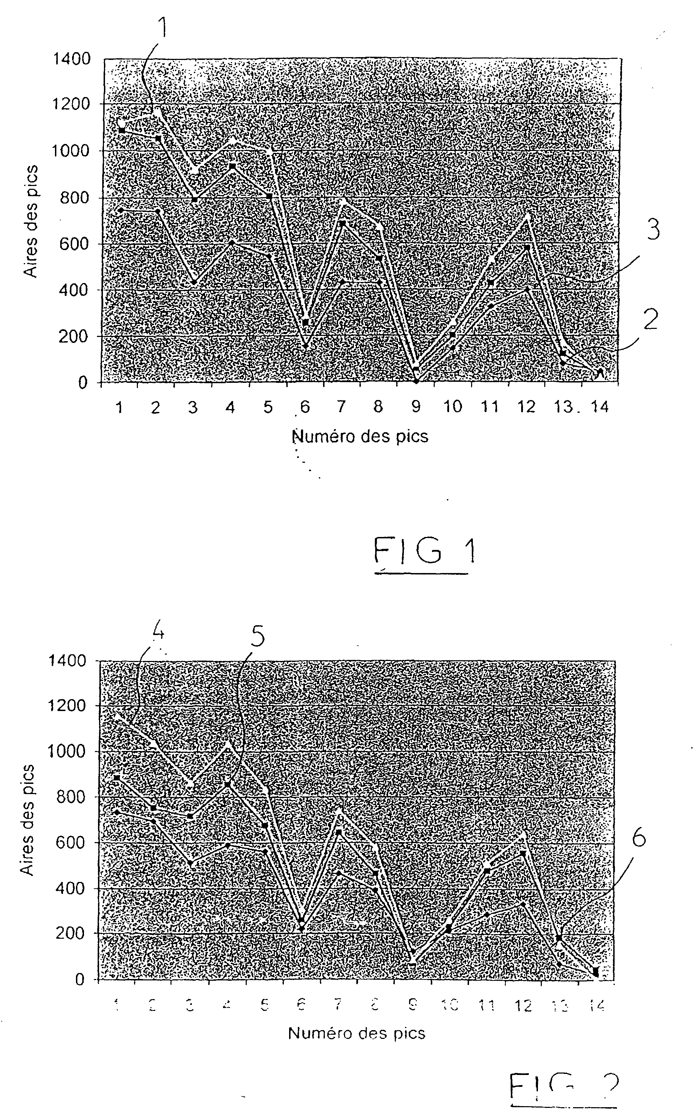

On a représenté à la figure 1, les résultats d'un chromatogramme effectué en HPLC de l'extrait final (1) par rapport à celui du fruit (2) et de la pulpe d'origine (3).

Comme le montre ce chromatogramme, le profil des anthocyanosides extraits est identique à celui des anthocyanosides natifs présents dans la pulpe ou dans le fruit d'origine, laissant présager ainsi que les caractéristiques physico-chimiques des anthocyanosides ne sont pas altérées tout au long du procédé d'extraction et de purification.

On peut donc penser que l'extrait obtenu, dont la composition en anthocyanosides est identique à celle présente dans le fruit originel garde toutes les qualités reconnues du fruit.

### Exemple 2

On reproduit l'exemple 1 à partir d'un second lot de myrtilles d'origine polonaise.

On observe que la concentration d'anthocyanosides obtenue est égale à 49,1 % en poids de l'extrait final, soit 52,01 % en poids de matière sèche.

La quantité résiduelle de solvants est :
- 0 ppm pour le butanol,
- inférieure à 3000 ppm pour le méthanol,
- inférieure à 2000 ppm pour l'éthanol.

De même que précédemment, la figure 2 représente le chromatogramme HPLC de la pulpe du fruit et de l'extrait obtenu.

On observe de façon identique que le profil des anthocyanosides contenus dans l'extrait (4) est identique à celui contenu dans la pulpe (5) et le fruit (6).

### Exemple comparatif 3

Dans cet exemple, on a comparé différentes caractéristiques d'extrait de myrtilles du commerce, respectivement :
* un extrait de myrtille commercialisé par la société INDENA dénommé ANTHOCYANOSIDE ;
* et un second extrait de myrtille commercialisé par la société VINYALS dénommé BILBERRY DRY EXTRACT (25 % anthocyanosidine) ;
par rapport à un extrait fabriqué selon le procédé de l'invention.

Les critères retenus sont :
- la concentration en anthocyanosides calculée en HPLC par rapport à un étalon de chlorure de cyanidine ;
- la quantité résiduelle de solvant dans l'extrait final.

Les résultats sont répertoriés dans le tableau suivant.

| | **INDENA SPECIFICATION** | **VINYALS SPECIFICATION** | **INVENTION SPECIFICATION** | **INVENTION RESULTAT** |
|---|---|---|---|---|
| **Anthocyanosides** | 23.75-26.25 % | 23.75-26.25 % | 45-55 % | 51,80 % |
| **Solvants organiques résiduels** | ≤ 30 000 ppm | ≤ 30 000 ppm | ≤ 3 000 ppm | 1035 ppm |
| • **éthanol** | ≤ 30 000 ppm | ? | ≤ 2 000 ppm | 890 ppm |
| • **méthanol** | ≤ 300 ppm | ? | ≤ 300 ppm | 145 ppm |
| • **autres solvants** | **≤** 50 ppm | ? | ≤ 1 ppm | ≤ 1 ppm |
| **Profil Chromatographique** | non déterminé | non déterminé | Fruit d'origine | Superposition des deux profils |

Comme le montre le tableau, l'extrait obtenu selon le procédé de l'invention permet d'obtenir une concentration en anthocyanosides très élevée par rapport aux extraits du commerce.

On note également une quantité résiduelle de solvant organique très faible.
Enfin, alors que rien n'est indiqué dans les spécifications des extraits INDENA et VINYALS concernant le profil chromatographique, on observe que le profil chromatographique de l'extrait de l'invention peut être superposé à celui du fruit d'origine.

Les avantages de l'invention concernant la restitution dans l'extrait des anthocyanosides natifs des fruits ressortent bien de la description.

On notera en particulier l'utilisation de solvants classiques d'extraction, lesquels sont éliminés tout au long du procédé de sorte que les solvants résiduels ne sont qu'à l'état de traces dans l'extrait obtenu.

De même, le procédé ne nécessite pas l'utilisation d'adjuvants du type SO₂ aux solvants d'extraction ou de composés chimiques, dont l'élimination est très délicate et pose un certain nombre de problèmes environnementaux.

Par ailleurs, on note que les anthocyanosides ne sont pas dénaturés tout au long du procédé et conservent une composition identique à celle du fruit d'origine, permettant ainsi de sauvegarder la totalité de leur activité.

On souligne enfin la concentration en anthocyanosides de l'extrait obtenu qui est au voisinage de 50 % en poids de matière sèche.

## Revendications

1. Procédé de purification d'un extrait de fruit rouge contenant des anthocyanosides selon lequel :
- on reprend ledit extrait avec une solution aqueuse ;
- on refroidit ensuite l'extrait aqueux jusqu'à ce qu'il atteigne une température homogène inférieure à 15° C ;
- après filtration de l'extrait aqueux, on récupère le perméat obtenu que l'on charge sur une résine polymérique macroréticulée ;
- on rince ensuite la résine par de l'eau déminéralisée ;
- puis, on élue la résine obtenue avec une solution d'élution alcoolique ;
- enfin, on concentre puis on sèche l'éluat obtenu.

2. Procédé selon la revendication 1, **caractérisé en ce que** l'on refroidit l'extrait aqueux jusqu'à ce qu'il atteigne une température homogène inférieure à 10°C.

3. Procédé selon la revendication 1, **caractérisé en ce que** l'extrait de fruit rouge sur lequel est effectuée la purification est un extrait alcoolique obtenu selon les étapes suivantes :
- on sépare tout d'abord la pulpe des fruits rouges entiers ;
- on met ensuite ladite pulpe au contact d'une solution d'extraction alcoolique ;
- on sépare la phase solide de la phase liquide ;
- enfin, on évapore sous vide la majeure partie de l'alcool résiduel contenu dans le phase liquide, afin d'obtenir un concentrât alcoolique.

4. Procédé selon la revendication 3, **caractérisé** en que le solvant mis en oeuvre lors de l'extraction alcoolique est choisi dans le groupe comprenant le méthanol, l'éthanol, le butanol, l'acétone.

5. Procédé selon l'une des revendications 1 ou 2, **caractérisé en ce que** la purification est effectuée sur un extrait prépurifié se présentant sous forme de poudre ou sous forme liquide.

6. Procédé selon l'une des revendications précédentes, **caractérisé en ce que** la solution d'élution alcoolique est une solution aqueuse d'éthanol dont la concentration en éthanol est comprise entre 10 et 90 %.

7. Procédé selon la revendication 5, **caractérisé en ce que** la solution d'élution alcoolique est une solution aqueuse d'éthanol dont la concentration en éthanol est égale à 40 %

8. Extrait purifié susceptible d'être obtenu selon le procédé objet de l'une des revendications 1 à 7.

9. Utilisation de l'extrait de la revendication 8 pour la préparation d'une composition pharmaceutique, cosmétique, diététique, nutraceutique, dans les alicaments, dans des aliments enrichis et les compléments alimentaires.

## Patentansprüche

1. Verfahren zur Reinigung eines Anthozyane enthaltenden Extrakts aus roten Früchten, wobei:
- der besagte Extrakt einer wässrigen Lösung zugesetzt wird;
- anschließend der wässrige Extrakt bis auf eine homogene Temperatur unter 15°C abgekühlt wird;
- nach dem Filtern des wässrigen Extrakts das erzielte Permeat gewonnen und auf ein zu Riesenmolekülen vernetztes Polymerharz geladen wird;
- das Harz anschließend mit entmineralisiertem Wasser gespült wird;
- das erzielte Harz mit einer alkoholischen Elutionslösung herausgelöst wird;
- das erzielte Eluat konzentriert und dann getrocknet wird.

2. Verfahren nach Anspruch 1, **dadurch gekennzeichnet, dass** man den wässrigen Extrakt bis auf eine homogene Temperatur unter 10°C abkühlen lässt.

3. Verfahren nach Anspruch 1, **dadurch gekennzeichnet, dass** der Extrakt aus roten Früchten, an dem die Reinigung vorgenommen wird, ein alkoholischer Extrakt ist, der in folgenden Schritten erzielt wird:
- man trennt zunächst das Fruchtfleisch von den ganzen roten Früchten;
- man bringt dann das Fruchtfleisch in Kontakt mit einer alkoholischen Extraktionslösung;
- man trennt die Festphase von der Flüssigphase;
- man lässt abschließend den Hauptanteil des in der Flüssigphase enthaltenen Restalkohols im Vakuum verdampfen, um ein alkoholisches Konzentrat zu erzielen.

4. Verfahren nach Anspruch 3, **dadurch gekennzeichnet, dass** das bei der alkoholischen Extraktion eingesetzte Lösungsmittel aus der Gruppe ausgewählt wird, zu der Methanol, Ethanol, Butanol, Aceton gehören.

5. Verfahren nach Anspruch 1 oder 2, **dadurch gekennzeichnet, dass** die Reinigung an einem vorgereinigten Extrakt erfolgt, der in Pulverform oder in flüssiger Form vorliegt.

6. Verfahren nach einem der vorhergehenden Ansprüche, **dadurch gekennzeichnet, dass** die alkoholische Elutionslösung eine wässrige Ethanollösung ist, wobei die Ethanolkonzentration zwischen 10 und 90% liegt.

7. Verfahren nach Anspruch 5, **dadurch gekennzeichnet, dass** die alkoholische Elutionslösung eine wässrige Ethanollösung ist, wobei die Ethanolkonzentration 40% beträgt.

8. Gereinigter Extrakt, der aus dem Verfahren nach einem der Ansprüche 1 - 7 hervorgegangen ist.

9. Verwendung des Extrakts nach Anspruch 8 für die Zubereitung einer pharmazeutischen, kosmetischen, diätetischen, nutrazeutischen Zusammensetzung in funktionellen Nahrungsmitteln, angereicherten Nahrungsmitteln und Nahrungsmittelzusätzen.

## Claims

1. Process for purifying a red fruit extract containing anthocyanosides, according to which:
- said extract is taken up in an aqueous solution;
- the aqueous extract is cooled until it reaches a homogeneous temperature of less than 15°C;
- after filtering the aqueous extract, the permeate obtained is recovered and loaded onto a macrocrosslinked polymeric resin;
- the resin is then rinsed with demineralized water;
- then the resin obtained is eluted with an alcoholic eluting solution;
- finally, the eluate obtained is concentrated and then dried.

2. Process according to Claim 1, **characterized in that** the aqueous extract is cooled until it reaches a homogeneous temperature of less than 10°C.

3. Process according to Claim 1, **characterized in that** the red fruit extract on which the purification is carried out is an alcoholic extract obtained according to the following steps:
- the pulp is first of all separated from the whole red fruits;
- said pulp is then brought into contact with an alcoholic extraction solution;
- then the solid phase is separated from the liquid phase;
- finally, the major portion of the residual alcohol contained in the liquid phase is evaporated under vacuum so as to obtain an alcoholic concentrate.

4. Process according to Claim 3, **characterized in that** the solvent used during the alcoholic extraction is chosen from the group comprising methanol, ethanol, butanol and acetone.

5. Process according to either of Claims 1 and 2, **characterized in that** the purification is carried out on a prepurified extract provided in powdered form or in liquid form.

6. Process according to one of the preceding claims, **characterized in that** the alcoholic eluting solution is an aqueous solution of ethanol whose ethanol concentration is between 10 and 90%.

7. Process according to Claim 5, **characterized in that** the alcoholic eluting solution is an aqueous solution of ethanol whose ethanol concentration is equal to 40%.

8. Purified extract which may be obtained according to the process which is the subject of Claims 1 to 7.

9. Use of the extract of Claim 8 for the preparation of a pharmaceutical, cosmetic, dietetic or nutraceutical composition, in functional foods, in enriched foods and dietary supplements.
